(19) Europäisches Patentamt
European Patent Office
Office européen des brevets

(11) Veröffentlichungsnummer: **0 048 914**
**A1**

(12) # EUROPÄISCHE PATENTANMELDUNG

(21) Anmeldenummer: **81107453.3**

(22) Anmeldetag: **19.09.81**

(51) Int. Cl.³: **C 07 C 33/40**, C 07 C 69/76, C 07 C 29/136, C 07 C 67/08

(30) Priorität: **01.10.80 DE 3036967**

(43) Veröffentlichungstag der Anmeldung: **07.04.82**
**Patentblatt 82/14**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Priesnitz, Uwe, Dr., Severinstrasse 58, D-5650 Solingen (DE)**
Erfinder: **Riebel, Hans-Jochem, Dr., In der Beek 92, D-5600 Wuppertal 1 (DE)**
Erfinder: **Fuchs, Rainer, Dr., Roeberstrasse 8, D-5600 Wuppertal 1 (DE)**
Erfinder: **Klauke, Erich, Dr., Eichendorffweg 8, D-5063 Odenthal (DE)**

(54) **Verfahren zur Herstellung von 3-Brom-4-fluor-benzylalkohol und neue Zwischenprodukte hierfür.**

(57) Die Erfindung betrifft ein Verfahren zur Herstellung von 3-Brom-4-fluor-benzylalkohol, das dadurch gekennzeichnet ist, daß man 3-Brom-4-fluor-benzoesäure(-derivate) mit Hydridkomplexen gegebenenfalls in Gegenwart von Katalysatoren und von Verdünnungsmitteln bei Temperaturen zwischen – 20 und + 150°C umsetzt oder 3-Brom-4-fluor-benzoesäure zunächst mit einem Chlorameisensäureester gegebenenfalls in Gegenwart eines Säureakzeptors und eines Verdünnungsmittels bei Temperaturen zwischen – 20 und + 50°C umsetzt und die hierbei gebildeten gemischten Anhydride mit Hydridkomplexen der Formel (III) gegebenenfalls in Gegenwart von Katalysatoren und von Verdünnungsmitteln bei Temperaturen zwischen – 20 und + 150°C umsetzt, sowie neue Zwischenprodukte dafür und Verfahren zu deren Herstellung.

EP 0 048 914 A1

BAYER AKTIENGESELLSCHAFT 5090 Leverkusen, Bayerwerk
Zentralbereich Rt/W
Patente, Marken und Lizenzen IVa/ZP

Verfahren zur Herstellung von 3-Brom-4-fluor-benzylalkohol und neue Zwischenprodukte hierfür

Die Erfindung betrifft ein neues Verfahren zur Herstellung von 3-Brom-4-fluor-benzylalkohol und neue Zwischenprodukte hierfür.

3-Brom-4-fluorbenzylalkohol und seine Herstellung sind Gegenstand einer eigenen älteren nicht-vorveröffentlichten Patentanmeldung. Die Herstellung von 3-Brom-4-fluor-benzylalkohol, erfolgt danach durch Umsetzung von 3-Brom-4-fluor-benzoylfluorid mit einem Hydridkomplex, wie z.B. Natriumboranat, in Gegenwart eines Verdünnungsmittels, wie z.B. iso-Propanol, bei Temperaturen zwischen 0 und 50°C (vgl. deutsche Patentanmeldung P 29 33 985 / Le A 19 875).

Es wurde ein Verfahren zur Herstellung von 3-Brom-4-fluor-benzylalkohol der Formel I gefunden,

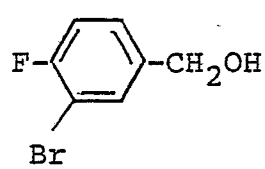 (I)

das dadurch gekennzeichnet ist, daß man

(a) 3-Brom-4-fluor-benzoesäure(-derivate) der Formel II

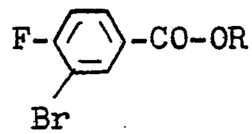

(II)

in welcher

R für Wasserstoff, Alkyl oder Alkoxycarbonyl steht,

mit Hydridkomplexen der Formel III

$M(M'H_4)$ (III)

in welcher

M für Lithium, Natrium oder Kalium steht und

M' für Bor oder Aluminium steht,

gegebenenfalls in Gegenwart von Katalysatoren und von Verdünnungsmitteln bei Temperaturen zwischen -2o und +15o°C umsetzt, oder

(b) 3-Brom-4-fluor-benzoesäure der Formel IIa

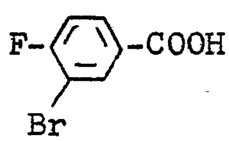

(II a)

zunächst mit einem Chlorameisensäureester der Formel IV

$Cl-CO-OR^1$ (IV)

in welcher

$R^1$ für Alkyl steht,

gegebenenfalls in Gegenwart eines Säureakzeptors und eines Verdünnungsmittels bei Temperaturen zwischen -2o und +5o $^{\circ}C$ umsetzt und die hierbei gebildeten gemischten Anhydride der Formel II b

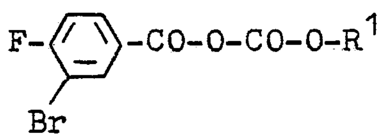

(II b)

in welcher

$R^1$ die oben angegebene Bedeutung hat,

mit Hydridkomplexen der Formel (III) gegebenenfalls in Gegenwart von Katalysatoren und von Verdünnungsmitteln bei Temperaturen zwischen -20 und +150 $^{\circ}C$ umsetzt.

Es wurden ferner die neuen 3-Brom-4-fluor-benzoesäure-derivate der Formel II c gefunden,

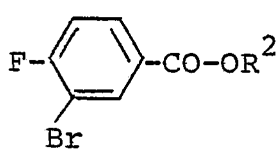

(IIc)

in welcher

$R^2$ für Alkyl oder Alkoxycarbonyl steht.

Man erhält die neuen Zwischenprodukte der Formel (II c), wenn man 3-Brom-4-fluor-benzoesäure (II a) oder deren reaktionsfähige Derivate mit Alkoholen bzw. Chlorameisensäureestern gegebenenfalls in Gegenwart von Katalysatoren, Säureakzeptoren und Verdünnungsmitteln umsetzt.

Überraschenderweise kann nach dem erfindungsgemäßen Verfahren 3-Brom-4-fluor-benzylalkohol aus 3-Brom-4-fluor-benzoesäure und ihren Derivaten der Formel (II) in sehr guten Ausbeuten und in hoher Reinheit hergestellt werden.

Vorteile des neuen Verfahrens liegen darin, daß die Ausgangsverbindung 3-Brom-4-fluor-benzoesäure in einfachen Verfahren mit hoher Ausbeute erhalten werden kann und daß das neue Verfahren ebenfalls bei geringem technischem Aufwand unter Verwendung kostengünstiger Hilfsmittel und Reaktionskomponenten durchzuführen ist und hohe Ausbeuten ergibt.

Die beim erfindungsgemäßen Verfahren ablaufenden Reaktionen können beispielsweise bei Verwendung von 3-Brom-4-fluor-benzoesäure-ethylester und Lithiumalanat (a) bzw. vom gemischten Anhydrid aus 3-Brom-4--fluor-benzoesäure und Kohlensäuremethylester sowie von Kaliumboranat (b) durch folgendes Formelschema skizziert werden:

(a)

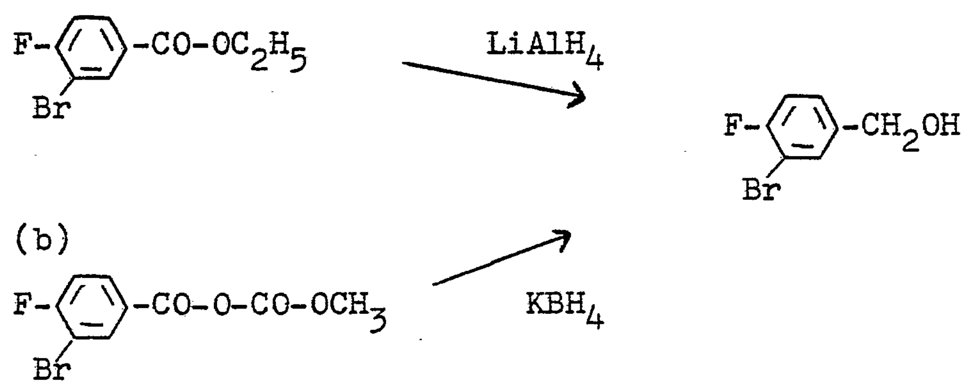


(b)

Die oben dargelegte Variante (a) des erfindungsgemäßen Verfahrens wird vorzugsweise unter Verwendung von Verdünnungsmitteln durchgeführt. Als solche kommen insbesondere Ether in Frage, wie z.B. Diethyl-, Dipropyl- und Dibutyl-ether, Glycoldimethylether und Diglycoldimethylether (Diglyme), sowie Tetrahydrofuran und Dioxan.

Man kann das erfindungsgemäße Verfahren auch in einem Zweiphasensystem aus Wasser und einem der oben genannten Ether durchführen. Hierbei werden vorzugsweise Phasentransferkatalysatoren verwendet. Beispiele hierfür sind Triethylbenzylammoniumbromid (TEBA), Tetrabutylammoniumbromid, Benzyl-triethylammoniumhydrogensulfat und Methyltrioctyl-ammoniumchlorid (Aliquat 336).

Bei Arbeiten in wasserfreien Lösungsmitteln werden vorzugsweise Katalysatoren aus der Reihe der sogenannten Lewis-Säuren, wie z.B. Bortrifluorid, Zinkchlorid oder Aluminiumchlorid verwendet. Letzteres wird besonders bevorzugt.

Hydridkomplexe der Formel (III), welche beim erfindungsgemäßen Verfahren verwendet werden können, sind z.B. Lithiumalanat (Lithium-tetrahydridoaluminat) und Natriumboranat (Natriumtetrahydridoborat). Letzteres wird besonders bevorzugt.

Die Variante (a) des erfindungsgemäßen Verfahrens wird bei Temperaturen zwischen -2o und +15o°C, vorzugsweise zwischen 0 und 1oo°C und gewöhnlich bei Normaldruck, d.h. zwischen o,1 und 1o bar, vorzugsweise zwischen o,5 und 5 bar, durchgeführt.

Je Mol 3-Brom-4-fluor-benzoesäure-(derivat) der Formel (II) werden zwischen o,5 und 2 Mol, vorzugsweise zwischen o,6 und 1,5 Mol Hydridkomplex der Formel (III) und zwischen o,1 und 1 Mol, vorzugsweise zwischen o,2 und o,5 Mol Katalysator eingesetzt.

In einer bevorzugten Ausführungsform der Verfahrensvariante (a) wird die Ausgangsverbindung der Formel (II) in einem der oben angegebenen Verdünnungsmittel vorgelegt und hierzu werden nacheinander oder gleichzeitig der Hydridkomplex der Formel (III) und der Katalysator gegeben. Das Reaktionsgemisch wird bis zum Ende der Umsetzung gerührt.

Die Aufarbeitung kann nach üblichen Methoden durchgeführt werden. Beispielsweise wird mit (Eis-)Wasser verdünnt und - z.B. mit Salzsäure - angesäuert. Hierbei scheidet sich der 3-Brom-4-fluor-benzylalkohol als öliges Rohprodukt ab, das durch Vakuumdestillation gereinigt werden kann. Bei einer anderen Aufarbeitungsvariante wird nach dem Ansäuern das Rohprodukt mit einem mit Wasser praktisch nicht mischbaren organischen Lösungsmittel, wie z.B. Methyl-tert.-butyl-ether, extrahiert und nach Waschen und Trocknen der organischen Pase durch Destillation isoliert.

Die bei der Verfahrensvariante (b) als Reaktionskomponenten zu verwendenden Chlorameisensäureester sind durch die Formel (IV) definiert. Vorzugsweise steht darin $R^1$ für $C_1$-$C_4$-Alkyl, insbesondere für Methyl oder Ethyl.

Als Beispiele seien Chlorameisensäuremethylester und Chlorameisensäurethylester genannt.

Die oben dargelegte Variante (b) des erfindungsgemäßen Verfahrens wird vorzugsweise unter Verwendung von Verdünnungsmitteln durchgeführt, wie sie oben für Variante (a) angegeben sind.

Die erste Stufe der Verfahrensvariante (b) wird vorzugsweise in Gegenwart von Säureakzeptoren durchgeführt. Als solche können praktisch alle üblichen Säurebindemittel Verwendung finden. Besonders bewährt haben sich Amine, wie z.B. Trimethylamin, Triethylamin, Dimethylanilin, Dimethylbenzylamin, Pyridin, Diazabicyclononen und Diazabicycloundecen.

Die Reaktionstemperatur wird in der ersten Stufe der Verfahrensvariante (b) zwischen -2o und +5o°C, vorzugsweise zwischen 0 und 3o°C gehalten. Das Verfahren wird im allgemeinen bei Normaldruck durchgeführt.

Je Mol 3-Brom-4-fluor-benzoesäure werden zwischen o,8 und 1,5 Mol, vorzugsweise zwischen o,95 und 1,2 Mol Chlorameisensäureester der Formel (IV) und zwischen o,8 und 1,5 Mol, vorzugsweise zwischen o,95 und 1,2 Mol Säurebindemittel eingesetzt.

In einer bevorzugten Ausführungsform der Verfahrensvariante (b) wird die 3-Brom-4-fluor-benzoesäure zusammen mit dem Säurebindemittel in einem der oben angegebenen Verdünnungsmittel vorgelegt und der Chlorameisensäureester der Formel (IV) wird langsam dazu gegeben.

Das Reaktionsgemisch wird bis zum Ende der Umsetzung gerührt und dann, gegebenenfalls unter erhöhtem oder

vermindertem Druck filtriert. Aus dem Filtrat kann das Intermediat der Formel (II b) nach üblichen Methoden isoliert werden. Vorzugsweise wird jedoch das Filtrat zur weiteren Umsetzung verwendet: Man gibt z.B. einen Phasentransferkatalysator dazu und versetzt langsam mit einer wässrigen Lösung von Natriumboranat. Nach Ende der Umsetzung wird angesäuert, die organische Phase abgetrennt, gewaschen, getrocknet, filtriert und eingeengt, wobei man als Rückstand den 3-Brom-4-fluor-benzylalkohol als farbloses Öl erhält.

Das Filtrat, welches die Verbindung der Formel (II b) enthält, kann alternativ auch wie oben für Variante (a) beschrieben umgesetzt werden.

Die 3-Brom-4-fluor-benzoesäure-derivate der Formel II c (oben) sind noch nicht in der Literatur beschrieben. In Formel (II c) steht $R^2$ vorzugsweise für $C_1$-$C_4$-Alkyl oder für $C_1$-$C_4$-Alkoxy-carbonyl.
Als Beispiele seien genannt:
3-Brom-4-fluor-benzoesäure-methylester, -ethylester, -n- und -iso-propylester, -n-, -iso-, sec.- und -tert.-butylester sowie 3-Brom-4-fluor-benzoesäure-kohlensäuremethylester-anhydrid und 3-Brom-4-fluor-benzoesäure-kohlensäure-ethylester-anhydrid.

Man erhält die Verbindungen der Formel (II c), in welcher $R^2$ für Alkoxycarbonyl steht, durch Umsetzung von 3-Brom-4-fluor-benzoesäure mit Chlorameisensäureestern der Formel (IV), gegebenenfalls in Gegenwart eines Säureakzeptors, wie z.B. Dimethylbenzylamin, und gegebenenfalls unter Verwendung eines Verdünnungsmittels, wie z.B. Tetrachlorkohlenstoff, bei Temperaturen zwischen -2o und +5o°C (vgl. Verfahrensvariante (b) oben). Die Produkte der Formel (II c) kann man isolieren, indem man nach Ende der Umsetzung filtriert und vom Filtrat das Lösungsmittel abdestilliert.

Die 3-Brom-4-fluor-benzoesäure-alkylester der Formel (IIc) erhält man, wenn man beispielsweise (a) 3-Brom-4-fluor-benzoesäure mit einem entsprechenden Alkohol gegebenenfalls in Gegenwart eines Katalysators, wie z.B. Schwefelsäure, umsetzt oder
(b) ein reaktionsfähiges Derivat, wie z.B. 3-Brom-4-fluor-benzoylfluorid, mit einem entsprechenden Alkohol, gegebenenfalls in Gegenwart eines Katalysators, wie z.B. Pyridin umsetzt.

Die Reaktionstemperaturen liegen hierbei jeweils zwischen 2o und 1oo°C.

Die Aufarbeitung kann nach beiden Varianten auf übliche Weise durchgeführt werden, beispielsweise durch Destillation des Reaktionsgemisches nach der Umsetzung. Man kann auch nach Abdestillieren des Alkohols zunächst den Rückstand in einem mit Wasser nicht mischbaren Lösungsmittel, wie z.B. Toluol, aufnehmen, die Lösung mit Wasser waschen, trocknen, filtrieren und dann destillieren.

Der nach dem erfindungsgemäßen Verfahren herzustellende 3-Brom-4-fluor-benzylalkohol kann als Zwischenprodukt zur Herstellung von Insektiziden verwendet werden (vgl. vorgängige Patentanmeldung P 2 933 985/Le A 19 875).

Hierzu wird 3-Brom-4-fluor-benzylalkohol zunächst durch Umsetzung mit Benzylchlorid in Gegenwart einer Base, wie z.B. Kalium-tert.-butylat, und eines Verdünnungsmittels, wie z.B. Tetrahydrofuran, bei Temperaturen zwischen 2o und 8o°C veräthert; der auf diese Weise erhaltene (3-Brom-4-fluor-benzyl)-benzyl-ether wird mit einem Phenolat, wie z.B. Natriumphenolat, in Gegenwart eines Katalysators, wie z.B. Kupfer(I)oxid, und eines Verdünnungsmittels, wie z.B. Isochinolin, bei Temperaturen zwischen 14o und 18o°C umgesetzt und der so erhaltene (4-Fluor-3-phenoxy-benzyl)-benzyl-ether nach bekannten Etherspaltungsmethoden, beispielsweise durch Erhitzen mit Bromwasserstoffsäure in 4-Fluor-3-phenoxy-benzylbromid umgewandelt.

Letzteres ist bereits als Zwischenprodukt für Insektizide bekannt (vgl. DE-OS 2 7o9 264).

Beispiel 1:

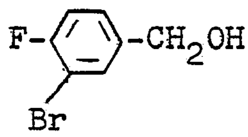

Reduktion von 4-Fluor-3-brombenzoesäure:

Zu einer Lösung von 1o,9 g (95%ig) 3-Brom-4-fluorbenzoesäure in 15o ml Diglyme werden bei 2o°C 6,7 g Aluminiumchlorid gegeben. Man rührt 1o Minuten bei 2o°C nach und gibt dann bei 2o-25°C portionsweise 5,7 g Natriumborhydrid zum Reaktionsgemisch. Nach 12 Stunden werden ca. 1oo ml Wasser (heftiges Schäumen) und anschließend ca. 1 ml 1o%ige Salzsäure zugetropft. Es werden ca. 1oo ml tert.-Butylmethylether hinzugefügt und die organische Phase wird abgetrennt. Sie wird einmal mit ca. 3o ml gesättigter Natriumbicarbonatlösung gewaschen. Anschließend wird die Etherphase eingeengt. Man erhält 6,7 g (76% der Theorie) 4-Fluor-3-brom-benzylalkohol.

Die Natriumbicarbonatlösung wird angesäuert. Es fällt 1 g nicht umgesetzte 4-Fluor-3-brombenzoesäure aus, die abgesaugt und an der Luft getrocknet wird.
Nach dem $^{1}$H-Kernresonanzspektrum hat die wiedergewonnene Säure die gleiche Qualität wie die eingesetzte Säure.

Beispiel 2:

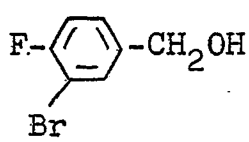

Zu einer Lösung von 2o,8 g 4-Fluor-3-brom-benzoesäure (95%ig) und 13,5 g Dimethylbenzylamin in 2oo ml Diethylether werden bei 0°C 1o,8 g Chlorameisensäureethylester

getropft. Man rührt 1 Stunde bei 0-2o$^{o}$C weiter und saugt dann das ausgefallene Hydrochlorid ab. Zum Filtrat tropft man bei 2o$^{o}$C nach Zugabe von o,1 g Triethylbenzylammoniumbromid eine Lösung von 7,6 g Natriumborhydrid in 5o ml Wasser. Man rührt 3 Stunden bei 2o$^{o}$C nach und säuert dann das Reaktionsgemisch mit verdünnter Salzsäure an. Die Etherphase wird abgetrennt und einmal mit 3o ml gesättigter Natriumbicarbonatlösung gewaschen. Anschließend wird die Etherphase eingeengt. Man erhält so 17,3 g (93,4 % der Theorie) 4-Fluor-3-brombenzylalkohol in Form eines farblosen Öles.

Die Natriumbicarbonatlösung wird angesäuert. Es fällt die nicht umgesetzte 4-Fluor-3-brombenzoesäure aus, die abgesaugt und an der Luft getrocknet wird. Man erhält 1 g 4-Fluor-3-brom-benzoesäure, die nach dem $^{1}$H-Kernresonanzspektrum die gleiche Qualität wie die eingesetzte Säure hat.

Die Reaktion läßt sich statt in Diethylether auch in Methyl-t-butylether durchführen.

<u>Beispiel 3</u>: 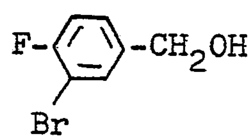

4-Fluor-3-brom-benzylalkohol aus Fluorbrombenzoesäuremethylester:

Eine Lösung von 23,3 g (o,1 Mol) 4-Fluor-3-brombenzoesäuremethylester in 5o ml Diglyme wird bei 5$^{o}$C portionsweise erst mit 3,8 g (o,1 Mol) Natriumborhydrid, dann mit 4,5 g (o,o33 Mol) Aluminiumchlorid versetzt. Man rührt ohne Kühlung bis zum Ende der exothermen Reaktion nach (Temperaturanstieg bis ca. 5o$^{o}$C) und läßt dann 1 Stunde bei 1oo$^{o}$C ausreagieren. Danach wird die Reaktionslösung

auf eine Mischung aus 125 ml Eiswasser und 12 ml konz. Salzsäure gegeben und der abgeschiedene Benzylalkohol abgetrennt und im Vakuum destilliert. Man erhält auf diese Weise 18,7 g (91% der Theorie) 4-Fluor-3-brom-benzylalkohol als farbloses Öl mit dem Siedepunkt 65-70°C (0,1 mbar).

"Eintopf-Verfahren"

Zu einer Lösung von 4,2 g 3-Brom-4-fluor-benzoesäure und 1,95 g Chlorameisensäuremethylester in 3o ml Tetrahydrofuran tropft man bei 2o°C unter Rühren 2 g Triethylamin gelöst in 5 ml Tetrahydrofuran. Man rührt noch 4o Minuten bei 2o-25°C weiter und saugt dann das ausgefallene Hydrochlorid ab. Zum Filtrat tropft man bei 1o-2o°C 5,5 g Natriumborhydrid gelöst in 2o ml Wasser unter Rühren zu. Anschließend wird noch 4 Stunden bei 2o°C nachgerührt. Der Reaktionsansatz wird dann in 1oo ml Wasser gegossen und mit 8o ml Methylenchlorid ausgeschüttelt. Die Methylenchloridphase wird abgetrennt, über Magnesiumsulfat getrocknet und dann das Lösungsmittel im Vakuum abdestilliert. Man erhält 1,95 g (95% der Theorie) 3-Brom-4-fluorbenzylalkohol als farbloses Öl.

<u>Beispiel 4</u>:

Darstellung des gemischten Anhydrids aus 4-Fluor-3-brombenzoesäure und Kohlensäureester:

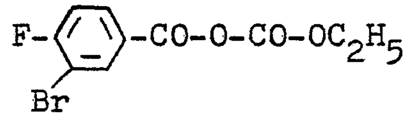

Zu einer Lösung von 1o,9 g (o,o5 Mol) 4-Fluor-3-brom-benzoesäure in 15o ml Tetrachlorkohlenstoff tropft man zuerst bei 0°C 7,6 g (o,o5 Mol) Dimethylbenzylamin und dann 5,4 g (o,o5 Mol) Chlorameisensäureethylester. Man rührt 3o Minuten bei 0°C nach und saugt dann vom entstandenen Hydrochlorid ab. Von der organischen Lösung wird ein Kernresonanzspektrum angefertigt. Das Kernresonanzspektrum beweist das Vorliegen der o.g. Verbindung.

Auf die gleiche Weise läßt sich aus Kohlensäuremethylesterchlorid und 4-Fluor-3-brom-benzoesäure auch das entsprechende Anhydrid der Formel

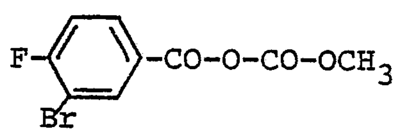

herstellen.

Beispiel 5

a) Darstellung von 4-Fluor-3-brombenzoesäuremethylester aus Fluorbrombenzoesäure:

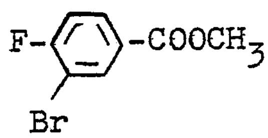

Eine Mischung aus 87,6 g (0,38 Mol) 95%iger 4-Fluor-3-brom-benzoesäure, 2oo ml Methanol und 3 ml Schwefelsäure wird 18 Stunden unter Rückfluß gekocht. Dann destilliert man das überschüssige Methanol im Vakuum ab, gibt 3oo ml Toluol zu und schüttelt die Lösung erst mit 5o ml Wasser, dann mit 5o ml gesättigter Natriumhydrogencarbonatlösung und anschließend wieder mit 5o ml Wasser. Danach destilliert man das Lösungsmittel im Vakuum ab, der Rückstand wird im Vakuum destilliert. Man erhält auf diese Weise 86 g (97% der Theorie) 4-Fluor-3-brombenzoesäuremethylester in Form eines farblosen, in der Kälte kristallin erstarrenden Öles mit dem Siedepunkt 64°C/0,1 Torr.

b) Darstellung aus Fluorbrombenzoylfluorid:

Zu einer Mischung aus 96 g (3 Mol) Methanol und 1 g Pyridin tropft man 221 g (1 Mol) 4-Fluor-3-brombenzoylfluorid. Anschließend kocht man das Reaktionsgemisch noch 2 Stunden unter Rückfluß und arbeitet dann durch Destillation auf. Man erhält so 217 g (93% der Theorie) 4-Fluor-3-brombenzoesäuremethylester in Form eines farblosen, in der Kälte erstarrenden Öles mit dem Siedepunkt 124°C/22 Torr (Schmelzpunkt 29-30°C).

Patentansprüche

1) Verfahren zur Herstellung von 3-Brom-4-fluor-benzylalkohol der Formel (I)

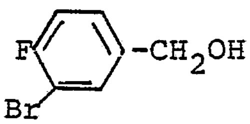

(I)

dadurch gekennzeichnet, daß man

a) 3-Brom-4-fluor-benzoesäure(-derivate) der Formel (II)

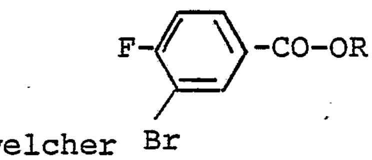

(II)

in welcher

R für Wasserstoff, Alkyl oder Alkoxycarbonyl steht,

mit Hydridkomplexen der Formel (III)

$$M(M'H_4) \qquad (III)$$

in welcher

M für Lithium, Natrium oder Kalium steht und
M' für Bor oder Aluminium steht,

gegebenenfalls in Gegenwart von Katalysatoren und von Verdünnungsmitteln bei Temperaturen zwischen -20 und +150°C umsetzt, oder

b) 3-Brom-4-fluor-benzoesäure der Formel (II a)

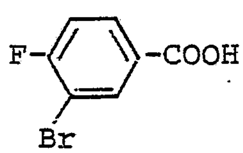

(II a)

zunächst mit einem Chlorameisensäureester der Formel (IV)

$$Cl-CO-OR^1 \qquad (IV)$$

in welcher

$R^1$ für Alkyl steht,

gegebenenfalls in Gegenwart eines Säureakzeptors und eines Verdünnungsmittels bei Temperaturen zwischen -20 und +50°C umsetzt und die hierbei gebildeten gemischten Anhydride der Formel (II b)

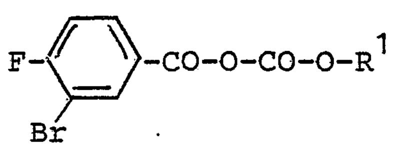

(II b)

in welcher

$R^1$ die oben angegebene Bedeutung hat,

mit Hydridkomplexen der Formel (III) gegebenenfalls in Gegenwart von Katalysatoren und von Verdünnungsmitteln bei Temperaturen zwischen -20 und +150°C umsetzt.

2) 3-Brom-4-fluor-benzoesäurederivate der Formel (II c)

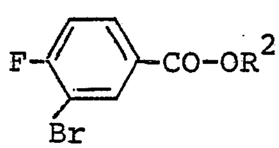

(II c)

in welcher

$R^2$ für Alkyl oder Alkoxycarbonyl steht.

3) Verfahren zur Herstellung von 3-Brom-4-fluor-benzoesäurederivaten der Formel (II c) (oben), dadurch gekennzeichnet, daß man 3-Brom-4-fluor-benzoesäure (II a) oder deren reaktionsfähige Derivate mit Alkoholen bzw. Chlorameisensäureestern gegebenenfalls in Gegenwart von Katalysatoren, Säureakzeptoren und Verdünnungsmitteln umsetzt.

Europäisches Patentamt

# EUROPÄISCHER RECHERCHENBERICHT

0048914 Nummer der Anmeldung

EP 81 10 7453.3

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 902 541 (SIGMA TAU)<br>* Anspruch 1 *<br>-- | 1 | C 07 C 33/40<br>C 07 C 69/76<br>C 07 C 29/136<br>C 07 C 67/08 |
| A,P | DE - A1 - 2 933 979 (BAYER)<br>* Seite 14, Zeilen 9 bis 21 *<br>-- | 1 | |
| A,P,<br>D | DE - A1 - 2 933 985 (BAYER)<br>* Anspruch 10 *<br>-- | 1 | |
| A | Chemical Abstracts Band 62, Nr. 2, 1965<br>Columbus, Ohio, USA<br>N.N. QUANG et al. "Orientation during the Friedel-Crafts acetylation of bromofluorobenzenes"<br>Spalte 1590 b-e<br>& Rec. Trav.Chim. Band 83, Nr. 9 bis 10, 1964, Seiten 1142 bis 1148<br>-- | 1 | RECHERCHIERTE SACHGEBIETE (Int. Cl.³)<br>C 07 C 29/00<br>C 07 C 29/136<br>C 07 C 33/40<br>C 07 C 67/00<br>C 07 C 67/08<br>C 07 C 69/76 |
| | Chemical Abstracts Band 65, Nr. 10, 1966<br>Columbus, Ohio, USA<br>J. PLESEK et al. "Chemistry of boranes. IV. Preparation, properties and behavior of magnesium borohydride towards Lewis bases"<br>Spalte 14809g-h bis 14810a-b<br>& Collection Czech. Chem. Commun. Band 31, Nr. 10, 1966, Seiten 3845 bis 3858<br>-- ./.. | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 22-12-1981 | KNAACK |

Europäisches Patentamt

**EUROPÄISCHER RECHERCHENBERICHT**

0048914 Nummer der Anmeldung

EP 81 10 7453.3

- Seite 2 -

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | Chemical Abstracts Band 71, Nr. 13, 1969 Columbus, Ohio, USA S. GIRI et al. "Search for new pesticides. Synthesis of some 3-aryl-2-(arylimino) thiazolidin-4-ones and related compounds" Seite 446, Abstract Nr. 61299s & Indian J. Appl. Chem. Band 31, Nr. 5 bis 6, 1968, Seiten 202 bis 206 -- | 2 | |
| A | Chemical Abstracts Band 72, Nr. 17, 1970 Columbus, Ohio, USA S. WATANABE et al. "Alcohols from hydrogenation of carboxylic acids or their esters" Seite 321, Abstract Nr. 89764j & JP - B - 70 - 00646 ---- | 1 | RECHERCHIERTE SACHGEBIETE (Int. Cl.³) |